Europäisches Patentamt

European Patent Office

Office européen des brevets

(19)

(11) Publication number: **0 336 670**
**A1**

# (12) EUROPEAN PATENT APPLICATION

(21) Application number: **89303245.8**

(22) Date of filing: **03.04.89**

(51) Int. Cl.⁴: **C07D 303/16 , //C08G59/22**

(30) Priority: **04.04.88 US 176955**

(43) Date of publication of application:
**11.10.89 Bulletin 89/41**

(84) Designated Contracting States:
**DE FR GB**

(71) Applicant: **HOECHST CELANESE CORPORATION**
**Route 202-206 North**
**Somerville, N.J. 08876(US)**

(72) Inventor: **Gupta, Balaram**
**54 Glen Court**
**North Plainfield New Jersey(US)**

(74) Representative: **De Minvielle-Devaux, Ian Benedict Peter et al**
**CARPMAELS & RANSFORD 43, Bloomsbury Square**
**London WC1A 2RA(GB)**

(54) **Diglycidyl muconate.**

(57) Diglycidyl muconate, a new composition of matter, is useful as a component in thermosetting resin systems, coating compositions and photoresist formulations.

EP 0 336 670 A1

## Diglycidyl Muconate

This invention provides a new polycarboxylic acid glycidyl ester of particular value.

Certain glycidyl esters of mono and polycarboxylic acids are known and some have found use in certain industrial applications. In U.S. Patent No. 2,448,602, glycidyl esters of lauric acid, palmitic acid, and rosin acids are disclosed. Glycidyl esters of dimers and trimers of long chain acids are described in U.S. Patent Nos. 2,772,296, 2,940,986 and 3,075,999. Other patents which disclose glycidyl ester compositions are: U.S. Patent No. 3,629,204 which is directed to glycidyl esters of aliphatic or alicyclic dicarboxylic acids; U.S. Patent No. 3,629,295, which describes glycidyl esters of sterically hindered organic acids; U.S. Patent No. 3,476,702, which is directed to curable compositions made from cycloaliphatic and cycloolefinic dibasic acids; and U.S. Patent No. 3,953,479 which claims polyglycidyl esters of $C_{21}$-cycloaliphatic acids. Additional glycidyl esters and processes for preparing them are disclosed in U.S. Patent No. 2,865,897; 3,576,827; and 3,859,314.

This invention is directed to a new substance the diglycidyl ester of muconic acid, that is, diglycidyl muconate.

Diglycidyl muconate is a white crystalline solid. In purified form it melts at about 110° C.

This diglycidyl ester can be crosslinked with conventional epoxy resin curing agents and will be useful in various systems where epoxy resins are useful. Diglycidyl muconate is particularly useful in curing systems which, in addition to conventional epoxy crosslinking agents, utilize radiant energy for the polymerization of the diene double bonds in the ester.

Diglycidyl muconate may be made from muconic acid and an epoxy derivatizing agent, e.g. epichlorohydrin or glycidol.

Muconic acid is a diolefinically unsaturated adipic acid derivative and can be made by the microbiological oxidation of various hydrocarbon substrates, as described in U.S. Patent Nos. 4,480,034 and 4,534,059.

In preparing the composition of this invention, muconic acid can be converted to muconoyl chloride by reaction with sulfurous oxychloride, zinc chloride and phosphorus trichloride, or phosphorus pentachloride. The muconoyl chloride may then be reacted with glycidol to form the diglycidyl compound.

Diglycidyl muconate can also be formed by reacting the sodium or potassium salt of muconic acid with epichlorohydrin, for example using the procedures described in U.S. Patent No. 3,859,314. Muconic acid may be reacted with epichlorohydrin in excess using a tertiary amine or quaternary ammonium compound as a catalyst, followed by dehydrohalogenation with caustic.

The following Examples illustrate the invention. Parts and percentages, unless otherwise indicated, are parts and percentages by weight.

### Example 1

To a suitable reactor are added 24.98 parts of muconic acid dichloride dissolved in 300 parts by volume of toluene. Agitation is begun and argon gas purge is applied. To an addition funnel there are added 24.42 parts of glycidol, 33.33 parts of triethylamine and 300 parts by volume of toluene. The resulting solution is then slowly added to the reactor while keeping the temperature in the reactor at 25° to 30° C. After the addition is completed, stirring is continued overnight. The reaction product is filtered and the solvent is removed from the filtrate using a rotary evaporator under high vacuum at a temperature no higher than 50° C. The resulting product has a melting point of 97° to 105° C. After recrystallization from methanol, analysis of the product by nuclear magnetic resonance and infra red shows the product to be diglycidyl muconate.

### Example 2

Using the same procedure described in Example 1, 25.62 parts of muconic acid dichloride are reacted with 25.40 parts of glycidol and 34.7 parts of triethylamine in 300 parts by volume of toluene. The recovered product, 23.9 parts, has a melting point of 94° to 100° C. The product is purified using column chromotography and chloroform as solvent. The impure product, 5 parts, is added to a 50 part silica gel column filled with chloroform. After 30 minutes absorption time, the product is eluted with chloroform and is collected in 250 parts by volume portions. The first three portions are roto evaporated. The crystalline diglycidyl muconate products have melting points of 110° C.

### Example 3

Using the procedure described in Example 1, 39.0 parts of muconic acid dichloride are reacted with 35.5 parts of glycidol and 48.42 parts triethylamine in 450 parts by volume of toluene. After stirring overnight at room temperature, the solution is filtered and the solvents are removed from the filtrate using a rotary evaporator and high vacuum at 50°C. 51.6 parts of product are recovered.

10 parts of the product are dissolved in 800 parts by volume of chloroform and 200 parts by volume of toluene. Silica gel, 50 parts, are added and are run through a glass column. The resulting product after removal of the solvents has a melting point range of 103° to 110°C.

Diglycidyl muconate can be utilized in a variety of applications. It can be cured with aliphatic and aromatic polyamines, with tertiary amines, with polycarboxylic acids and anhydrides, with Lewis acids, polymercaptans, carboxylic acid containing vinyl and acrylic copolymers, and the like.

Curing agents for epoxide resins can be prepared by reacting the diglycidyl muconate with polyamines, polymercaptans, and polycarboxylic acids using the coreactant in excess over the glycidyl groups so that the resulting curing agent contains the functional amine, mercaptan or acid group.

Diglycidyl muconate can be polymerized to intermediate and high molecular weight polymers by reaction with dicarboxylic acids, dihydric phenols, or dihydric alcohols. The intermediate molecular weight polymers can be modified by esterification with fatty acids to form air-drying and heat-curing coating compositions. The intermediate and high molecular weight polymers can also be cured by reaction with polyisocyanates, aminoplast resins, phenolplast resins and acid containing acrylic copolymers. The high molecular weight polymers can also be used without further modification as thermoplastic molding compositions.

Diglycidyl muconate is particularly useful in applications which use the reactivity of the glycidyl groups and the reactivity of the diene unsaturation. For example, diglycidyl muconate and phthalic anhydride when dissolved in styrene monomer which contains a peroxide catalyst, can be cured by heat to form highly crosslinked compositions.

Diglycidyl muconate or its derivatives are also useful in various radiation curing applications, e.g., ultraviolet or electron beam radiation. A particularly useful derivative is the diacrylic or methacrylic ester of diglycidyl muconate. Under radiation curing conditions as well as free-radical reactive conditions, this derivative is trifunctional and cures to highly crosslinked compositions.

The principles, preferred embodiments and modes of operation of the present invention have been described in the foregoing specification. The invention which is intended to be protected herein, however, is not to be construed as limited to the particular forms disclosed, since these are to be regarded as illustrating rather than restrictive. Variations and changes may be made by those skilled in the art without departing from the spirit of the invention.

## Claims

1. The diglycidyl ester of muconic acid.

2. A process for the production of diglycidyl muconate which comprises converting muconic acid to muconyl chloride by reaction with sulfurous oxychloride, zinc chloride and phosphorus trichloride or phosphorus pentachloride, and reacting the muconyl chloride with glycidol.

3. A process for the production of diglycidyl muconate which comprises reacting muconic acid on its rodium or potassium salt with epichlorohydrin in excess, using a tertiary amine or quaternary ammonium compound as catalyst, followed by dehydrohalogenation with caustic.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| D,Y | US-A-2 940 986  (H.A. NEWEY) <br> * Whole document * <br> --- | 1-3 | C 07 D 303/16 // <br> C 08 G  59/22 |
| Y | EP-A-0 028 024  (OKAMURA OIL MILL LTD) <br> * Whole document * <br> --- | 1-3 | |
| Y | CHEMICAL ABSTRACTS, vol. 67, no. 24, 11th December 1967, page 10299, abstract no. 109054e, Columbus, Ohio, US; I.A. ARBUZOVA et al.: "Polymeric esters of muconic acid", & VYSOKOMOL. SOEDIN., SER. B 9(8), 638-42 (1967) <br> * Abstract * <br> ----- | 1-3 | |
| | | | TECHNICAL FIELDS SEARCHED (Int. Cl.4) |
| | | | C 07 D 303/00 |

The present search report has been drawn up for all claims

| Place of search | Date of completion of the search | Examiner |
|---|---|---|
| THE HAGUE | 03-07-1989 | ALLARD M.S. |